# EUROPÄISCHE PATENTSCHRIFT

(11) **EP 0 632 706 B1**
(45) Veröffentlichungstag und Bekanntmachung des Hinweises auf die Patenterteilung: **10.05.2000**
(21) Anmeldenummer: 94903800.4
(22) Anmeldetag: 15.12.1993
(51) Int. Cl.: A61B 1/00, A61M 25/01

(54) **VORRICHTUNG ZUM BEWEGEN EINES ENDOSKOPS LÄNGS EINES KANALARTIGEN HOHLRAUMS**
DEVICE FOR MOVING AN ENDOSCOPE THROUGH A CHANNEL-LIKE CAVITY
DISPOSITIF PERMETTANT DE DEPLACER UN ENDOSCOPE A TRAVERS UNE CAVITE SIMILAIRE A UN CANAL

(30) Priorität: 15.12.1992 DE 4242291
(43) Veröffentlichungstag der Anmeldung: 11.01.1995
(60) Teilanmeldung: 99115752.0
(73) Patentinhaber: STM Medizintechnik Starnberg GmbH, 82319 Starnberg (DE)
(72) Erfinder: GRÜNDL, Andreas, D-81377 München (DE); BOB, Alexander, D-68163 Mannheim (DE); BOB, Konstantin, D-69469 Weinheim (DE)
(74) Vertreter: Tiedtke, Harro, Dipl.-Ing.
(86) Internationale Anmeldenummer: EP9303570
(87) Internationale Veröffentlichungsnummer: WO9413188

(56) Entgegenhaltungen:
- DE-C- 3 329 176
- FR-A- 1 456 623
- US-A- 4 321 915
- US-A- 5 163 927

## Beschreibung

Die Erfindung bezieht sich auf eine Vorrichtung zum Bewegen eines Endoskops längs eines kanalartigen Hohlraums gemäß dem Oberbegriff des Patentanspruchs 1.

Endoskope sind zu einem wichtigen Hilfsmittel in der Technik und in der Medizin geworden, um kanalartige Hohlräume zu inspizieren, die auf andere Weise nicht oder nur mit erheblichen Eingriffen zugänglich sind. Endoskope sind an ihrem Distalende mit einer Beleuchtungseinrichtung und mit einer Optik zur visuellen Erfassung des davorliegenden Bereichs des Hohlraums ausgerüstet. Die am Distalende des Endoskops erfaßte, optische Information wird normalerweise entweder mittels einer Faseroptik durch das Endoskop nach hinten zu seinem Bedienungsende übertragen oder wird mittels eines Kamerachips am Distalende erfaßt, durch eine elektrische Leitung durch das Endoskop nach hinten geleitet und auf einem Bildschirmmonitor sichtbar gemacht. Insgesamt haben Endoskope, abgesehen von dem hinteren Bedienungsende, üblicherweise eine langgestreckte, biegsam-stabförmige Gestalt.

Um einen kanalartigen Hohlraum inspizieren zu können, wird das Endoskop durch eine Zugangsöffnung in den Hohlraum eingebracht. Das weitere Hineinbewegen des Endoskops geschieht üblicherweise dadurch, daß eine Bedienungsperson an dem aus der Zugangsöffnung herausragenden Teil des Endoskops mit der Hand angreift und von dort her das drucksteife Endoskop allmählich immer weiter in den Hohlraum hineinschiebt.

Dieses zunehmende Hineinbewegen des Endoskops in den kanalartigen Hohlraum ist relativ mühsam. Besonders schwierig gestaltet sich das Vorschieben des Endoskops, wenn der zu inspizierende, kanalartige Hohlraum engere Biegungen, Engstellen oder dergleichen aufweist. Wenn der kanalartige Hohlraum eine nichtglatte Wand aus einem wenig festen Material aufweist, besteht die Gefahr, daß das Distalende beim Vorschieben an der Hohlraumwand hängen bleibt; dies kann zu Beschädigungen der Hohlraumwand führen, Insbesondere wenn der Hohlraum mehrere Biegungen aufweist, wird durch das Anschieben vom hinteren Ende des Endoskops her erheblicher Druck von innen her gegen die jeweils biegungsäußeren Wandbereiche ausgeübt. Das weitere Vorschieben des Endoskops ist erschwert.

Aus dem gattungsbildenden Stand der Technik gemäß der DE-PS-33 29 176 ist eine Endoskopvorrichtung bekannt, die einen Endoskopschaft hat, der von einem elastischen Stülpschlauch umgeben ist. Dieser Schlauch bildet an dem distalen Ende des Endoskopschafts eine Mehrfachumstülpung oder Faltung wodurch der Stülpschlauch in mehreren Lagen aufeinander geschichtet wird. Zur Vorwärtsbewegung des Endoskopschafts dient eine Druckbeaufschlagungseinrichtung, mittels der ein durch den Stülpschlauch gebildeter Hohlraum unter Druck gesetzt werden kann. Durch diesen Überdruck stülpt sich der Stülpschlauch in seinem vorderen Stülp- oder Faltbereich allmählich aus und nimmt dabei den Endoskopschaft aufgrund der Haftreibung zwischen dem Schlauch und Schaft mit.

Diese bekannte Endoskopvorrichtung hat jedoch den Nachteil, daß durch den Überdruck in dem Hohlraum des Stülpschlauchs die elastische Schlauchaußenwand radial aufgeweitet wird, wodurch der Wände des zu untersuchenden Kanals druckbeaufschlagt und ggf. ebenfalls aufgeweitet werden. Dies kann zu Komplikationen während der Fortbewegung des Endoskopschafts führen.

Der Erfindung liegt daher die Aufgabe zugrunde, einen Weg für ein komplikationsloseres Bewegen des Endoskops längs des Hohlraums mit verringerter Gefahr einer Beschädigung der Hohlraumwand verfügbar zu machen.

Diese Aufgabe wird durch eine Vorrichtung mit den Merkmals gemäß dem Patentanspruch 1 gelöst.

Aufgrund der erfindungsgernäßen Vorrichtung wird nicht nur der Endoskopschaft in den kanalartigen Hohlraum mit einer continuierlichen Bewegung eingebracht und längs diesem vorgeschoben, sondern es wird auch der Stülpschlauch zwischen der Hohlraumwand und dem Endoskop ohne die Gefahr einer radialen Aufweitung eingebracht. Der Stülpschlauch ergibt eine Auskleidung des Hohlraums, welche das Bewegen des Endoskopschafts längs des Hohlraums erleichtert und die Gefahr von Beschädigungen der Hohlraumwand entscheidend verringert.

Ein gemeinsames Vorwärtsbewegen des vorderen Teils des Endoskopschafts und des vorderen Teils des inneren Stülpschlauchbereichs führt normalerweise dazu, daß sich diese beiden vorderen Teile mit praktisch gleicher Geschwindigkeit vorwärtsbewegen. Da von einer bestimmten, vorgeschobenen Länge des inneren Stülpschlauchbereichs die Hälfte nach Durchwandern des Umstülpbereichs für den äußeren Stülpschlauchbereich "verbraucht" wird, ergäbe sich im Ergebnis, daß der vordere Teil des Endoskopschafts um diese halbe Länge nach vorn über den Umstülpbereich des Stülpschlauchs vorsteht. Um dies zu vermeiden, wird erfindungsgemäß der vordere Teil des Endoskopschafts mit einer Geschwindigkeit vorwärtsbewegt, die im wesentlichen halb so groß wie die Bewegungsgeschwindigkeit des vorderen Teils des inneren Stülpschlauchbereichs ist. Hiermit einher geht eine Relativ-Gleitbewegung des inneren Stülpschlauchbereichs relativ zu dem Endoskop.

Die erfindungsgemäße Möglichkeit zur Erreichung des genannten Geschwindigkeitsverhältnisses besteht darin, eine Vorwärts-Antriebskraft auf den vorderen Teil des Endoskopschafts und den vorderen Teil des inneren Bereichs des Stülpschlauchs auszuüben und gleichzeitig eine Rückwärts-Bewegungsbestimmungskraft auf einen hinten aus dem Stülpschlauch herausreichenden, hinteren Teil des Endoskopschafts auszuüben. Der erfindungsgemäße Bewegungsablauf der vorderen Teile des Endoskopschafts auszuüben.

Der erfindungsgemäße Bewegungsablauf der vorderen Teile des Endoskopschafts und des inneren Stülpschlauchbereichs kann grundsätzlich für die gesamte Vorwärtsbewegungsstrecke kontinuierlich durchgeführt werden. Man kann aber auch die Gesamtbewegungsstrecke auf mehrere, nacheinander mit Zeitabstand erfolgende Bewegungsschritte aufteilen.

Die Erfindung läßt die Möglichkeit, das Endoskop zu Zeiten, in denen keine Vorwärtsbewegung der vorderen Teile von Endoskopschaft und Stülpschlauch mit dem beschriebenen Geschwindigkeitsverhältnis stattfindet, relativ zu dem inneren Bereich des Stülpschlauchs zu verschieben, also einfach in der geschaffenen Auskleidung längs des Hohlraums zu bewegen. Eine typische Situation ist zum Beispiel, daß die Bedienungsperson sieht, daß sich vor dem Distalende des Endoskops ein ziemlich gerader Hohlraumabschnitt mit einigermaßen glatter Wand befindet. Dann kann die Bedienungsperson den Endoskopschaft relativ zu dem als Ganzes ruhenden Stülpschlauch; der Stülpschlauch kann dann anschließend durch Ziehen an seinem inneren Bereich aus dem Hohlraum herausbewegt werden.

In bevorzugter Weiterbildung der Erfindung kann man die Möglichkeit vorsehen, den Raum zwischen dem äußeren Bereich und dem inneren Bereich des Stülpschlauchs mittels eines Fluids mit Druck zu beaufschlagen. Als Fluid eignen sich Flüssigkeiten oder Gase. Infolge dieser Druckbeaufschlagung werden der äußere Bereich und der innere Bereich des Stülpschlauchs auseinanderbewegt oder auseinandergehalten, mit der Folge, daß die Reibung zwischen diesen beiden Bereichen minimiert wird. Der innere Bereich des Stülpschlauchs wird in Längsrichtung straff gehalten. Außerdem ergibt sich ein geringfügiger zusätzlicher Vortriebseffekt auf den inneren Bereich des Stülpschlauchs, weil der in dem genannten Raum herrschende Überdruck den Umstülpbereich des Stülpschlauchs nach vorn zu bewegen trachtet. Schließlich kann sich durch diesen Überdruck ein Anpressen des inneren Bereichs des Stülpschlauchs gegen den Endoskopschaft auf großer Länge ergeben, was einen mitziehenden Vortriebseffekt auf das Endoskop ergibt. Es versteht sich, daß der Raum zwischen dem äußeren Bereich und dem inneren Bereich des Stülpschlauchs an einer von dem Umstülpbereich beabstandeten Stelle abgeschlossen sein muß, um die beschriebene Druckbeaufschlagung zustandebringen zu können.

Ferner ist es möglich, das Fluid aus dem Raum zwischen dem äußeren Bereich und dem inneren Bereich des Stülpschlauchs abströmen zu lassen oder abzusaugen, woraufhin sich dieser Raum unter der Wirkung des äußeren Luftdrucks abflacht, also der innere Bereich des Stülpschlauchs von dem Endoskopschaft freikommt. Dies ist günstig insbesondere für Bewegungen des Endoskopschafts relativ zu dem insgesamt ruhenden Stülpschlauch.

Vorzugsweise befindet sich in dem Raum zwischen dem Endoskopschaft und dem inneren Bereich des Stülpschlauchs ein Schmiermittel, wodurch die Reibung zwischen diesen beiden Elementen bei ihren Relativbewegungen verringert wird. Man kann weiteres Schmiermittel kontinuierlich oder wiederholt in diesem Raum einpressen.

Man kann zu Beginn des Hineinbewegens des Endoskopschafts und des Stülpschlauchs in den Hohlraum und während des weiteren Verlaufs des Hineinbewegens eine Vorratslänge von Stülpschlauch und Endoskopschaft in geradliniger Lage vorsehen. Insbesondere bei einer beträchtlichen einzuführenden Länge des Endoskopschafts ist es jedoch aus Platzgründen häufig bevorzugt, daß ein Teil des Stülpschlauchs und - darin aufgenommen - ein Teil des Endoskopschafts in nichtgeradliniger Lage als beim Vorwärtsbewegen des vorderen Teils des Endoskopschafts längs des Hohlraums zu verringernder Vorrat vorgesehen ist, wobei ein hinterer Teil des Endoskopschafts hinten aus dem Vorratsteil des Stülpschlauchs herausreicht; und daß die Bewegungsbestimmungseinrichtung auf den Endoskopschaft in dessen hinteren Teil wirkt. Es kann ein Vorratsstraffungselement vorgesehen sein, das eine Gegenkraft gegen eine Begradigung des Vorrats ausübt;
Es sei an dieser Stelle betont, daß - je nach Aufbau der Vorrichtung - die Bewegungsbestimmungseinrichtung insbesondere eher die Funktion hat, die Vorwärts-Bewegungsgeschwindigkeit des Endoskopschafts hemmend auf die gewünschte Geschwindigkeit herabzusetzen, oder insbesondere eher die Funktion hat, den Endoskopschaft in Rückwärtsrichtung relativ zu dem hinteren Ende des Stülpschlauchs anzutreiben. Die Bewegungsbestimmungseinrichtung soll vorzugsweise dem Endoskopschaft eine vorbestimmte, definierte Geschwindigkeit mitteilen, damit das weiter vorn beschriebene Geschwindikgeitsverhältnis zwischen dem Endoskop und dem inneren Stülpschlauchbereich erreicht wird.

Eine als besonders einfach bevorzugte Art der Ausbildung der Bewegungsbestimmungseinrichtung besteht darin, ein Paar von elektromotorisch antreibbaren, auf das Endoskop arbeitenden Antriebsrädern vorzusehen.

Ferner kann man eine zusätzliche Antriebseinrichtung vorsehen, mit der sich das Endoskop relativ zu dem ruhenden, inneren Bereich des Stülpschlauchs bewegen läßt. Diese zusätzliche Antriebseinrichtung kann insbesondere ebenfalls von dem Paar von elektromotorisch antreibbaren, auf das Endoskop arbeitenden Antriebsrädern der Bewegungsbestimmungseinrichtung gebildet sein. Zu diesem Zweck können diese Antriebsräder wahlweise in beiden Drehrichtungen antreibbar sein.

Es gibt die Möglichkeit, den Stülpschlauch nicht nur vorne zur Ausbildung eines äußeren Bereichs und eines inneren Bereichs umzustülpen, sondern dies zusätzlich auch im hinteren Bereich, der sich beim Betrieb der Vorrichtung außerhalb des kanalartigen Hohlraums befindet, vorzusehen. Beim Hineinbewegen des Endoskopschafts in den Hohlraum wird dann am vorderen Ende der äußere, ruhende Bereich des Stülpschlauchs immer länger und wird am hinteren Ende der äußere, ruhende Bereich des Stülpschlauchs immer kürzer. Diese Ausbildung der erfindungsgemäßen Vorrichtung bietet Vorteile hinsichtlich der erforderlichen Länge des Endoskops, wie weiter unten bei der Beschreibung von Ausführungsbeispielen noch deutlicher werden wird.

Zur Reibungsverminderung kann der Stülpschlauch außenseitig (und damit auch innenseitig im umgestülpten, äußeren Bereich) und/oder innenseitig mit einem reibungsarmen Material, z. B. Polytetrafluorethylen, beschichtet sein.

Zum Rückwärts-Bewegen des Endoskops besteht eine erste Möglichkeit darin, einfach den Endoskopschaft in dem ruhenden, inneren Bereich des Stülpschlauchs zurückzuziehen und danach den Stülpschlauch aus dem Hohlraum durch Ziehen an seinem inneren Bereich herauszuholen. Eine zweite Möglichkeit besteht darin, umgekehrt zu verfahren wie beim Vorwärts-Bewegen, d. h. den Endoskopschaft mit im wesentlichen der halben Geschwindigkeit des inneren Bereichs des Stülpschlauchs rückwärts zu bewegen. Die Bewegungsbestimmungseinrichtung übt darm - in umgekehrter Richtung wie beim Vorwärts-Bewegen - eine Kraft auf den Endoskopschaft aus, die das beschriebene Geschwindigkeitsverhältnis sicherstellt. Diese Art der Rückwärts-Bewegung hat den Vorteil, daß der kanalartige Hohlraum ein zweites Mal inspiziert werden kann.

Die Erfindung läßt sich zum einen bei der Inspektion, aber auch bei der Durchführung von Arbeiten, in technischen Anlagen und Einrichtungen einsetzen. Als Beispiele unter vielen seien Kernreaktoren, chemische Anlagen, Rohrleitungssysteme, genannt. Zum anderen läßt sich die Erfindung günstig auf dem Gebiet der Medizin einsetzen, insbesondere zur Exploration von Hohlräumen oder röhrenartigen Kanälen des Körpers. Endoskope haben sich besonders eingeführt zur Exploration der Speiseröhre, des Magens, des Zwölffingerdarms vom Magen aus, des Darms vom Anus aus, der Harnröhre, der Blase und der Harnleiter. Mit Hilfe von Endoskopen lassen sich heute minimal invasive Operationstechniken durchführen. Endoskope weisen in der Regel einen sogenannten Arbeitskanal auf, durch den diverse Arbeitselemente eingeführt und bedient werden können, z. B. kleine Zangen zur Entnahme von Gewebsproben, Biopsienadeln, beheizbare Schneiddrähte, kleine Scheren, Koagulationselektroden oder dergleichen. Schließlich sind in der Regel ein Fluidkanal für Spülflüssigkeit und Bedienungsdrähte zum Abwinkeln des Distalendes des Endoskops in mehrere Richtungen vorhanden. Insgesamt soll der Begriff "Endoskop" der vorliegenden Anmeldung alle Arten von längs eines kanalartigen Hohlraums zu bewegenden Instrumenten oder Einrichtungen umfassen, auch wenn der Gesichtspunkt des optischen Inspizierens nicht im Vordergrund steht oder überhaupt kein optisches Inspizieren stattfindet.

Ferner gibt die Erfindung die Möglichkeit, zunächst mit einem Endoskop unter Sichtkontrolle eine bestimmte Stelle eines kanalartigen Hohlraums anzufahren, dann das Endoskop aus dem auskleidenden Stülpschlauch zurückzuziehen und anschließend ein anderes Instrument zur Durchführung einer bestimmten Aufgabe ganz unproblematisch durch den auskleidenden Stülpschlauch zu derjenigen Stelle, wo etwas getan werden soll, vorzuschieben. Als Beispiel sei ein Ballondilatationsinstrument genannt.

Besonders mühsam, schwierig und zeitraubend war bisher die Koloskopie, also die Exploration des Dickdarms vom Anus her. Der Darm weist Abbiegungen und häufig Engstellen auf. Demzufolge gehören koloskopische Untersuchungen bisher zu den aufwendigen und für den Patienten unangenehmen Untersuchungen und kommen deshalb für eine breite Anwendung, insbesondere im Sinn regelmäßiger Vorsorgeuntersuchungen, kaum in Betracht. Das Umgehen mit einem Koloskop erfordert einen hierin besonders erfahrenden Arzt und besonders erfahrenes Hilfspersonal.

Die Situation ist besonders nachteilig, weil Anomalien der Darmwand, beispielsweise Polypen, Adenome und Karzinome, in vielen Gegenden der Welt immer zahlreicher werden und weil eine möglichst frühzeitige Erkennung die Heilungsschancen für den betreffenden Patienten ganz erheblich erhöht bzw. zu einer erheblichen Lebensverlängerung führt.

Insofern ist es äußerst wünschenswert, ein Endoskop zur Verfügung zu haben, das unkomplizierter und risikoloser längs des Darms bewegbar ist und das auch von einschlägig nicht so erfahrenen Ärzten bedient werden kann.

Bisher mußte der Patient bei der Durchführung einer Koloskopie wiederholt umgelagert werden und mußte durch gerichtetes Drücken auf den Leib des Patienten versucht werden, die Darmbiegungen so weit zu begradigen, daß das Endoskop einigermaßen gut vorgeschoben werden konnte. Durch die Erfindung wird auf medizinischem Gebiet eine entscheidende Verbesserung gerade bei der Koloskopie erreicht, insbesondere weil durch die Auskleidung des Darms mit dem schützenden, glatt-führenden, äußeren Bereich des Stülpschlauchs die Gefahr der Beschädigung oder gar der Perforation der empfindlichen Darmwand wesentlich reduziert wird. Durch die Erfindung wird die totale Koloskopie bis hin zum Übergang vom Dickdarm in den Dünndarm für eine echte Breitenanwendung, beispielsweise im Sinne allgemeiner Vorsorgeuntersuchen in sinnvollen Zeitabständen ab einem bestimmten Lebensjahr, zugänglich. Es ergibt sich eine Personalersparnis bei den Untersuchungen.

Die Erfindung und Weiterbildung der Erfindung werden nachfolgend anhand eines teilweise schematisiert dargestellten Ausführungsbeispiels noch näher erläutert. Es zeigt:
Fig. 1 in schematisierter Form eine Vorrichtung zum Bewegen eines Endoskops längs des Darms;
Fig. 2 teilweise geschnitten eine zweite Antriebseinrichtung und eine Schmiermittel-Einpreßeinrichtung der Vorrichtung von Fig. 1;
Fig. 3 in schematisierter Form analog zu Fig. 1 eine bevorzugte Ausführungsform einer Vorrichtung zum Bewegen eines Endoskops längs des Darms;
Fig. 4 in schematisierter Form analog zu Fig. 1 eine Variante mit Vorratsstraffungselement;
Fig. 5 schematisiert das Distalende des Endoskops mit einem Relativlagesensor.

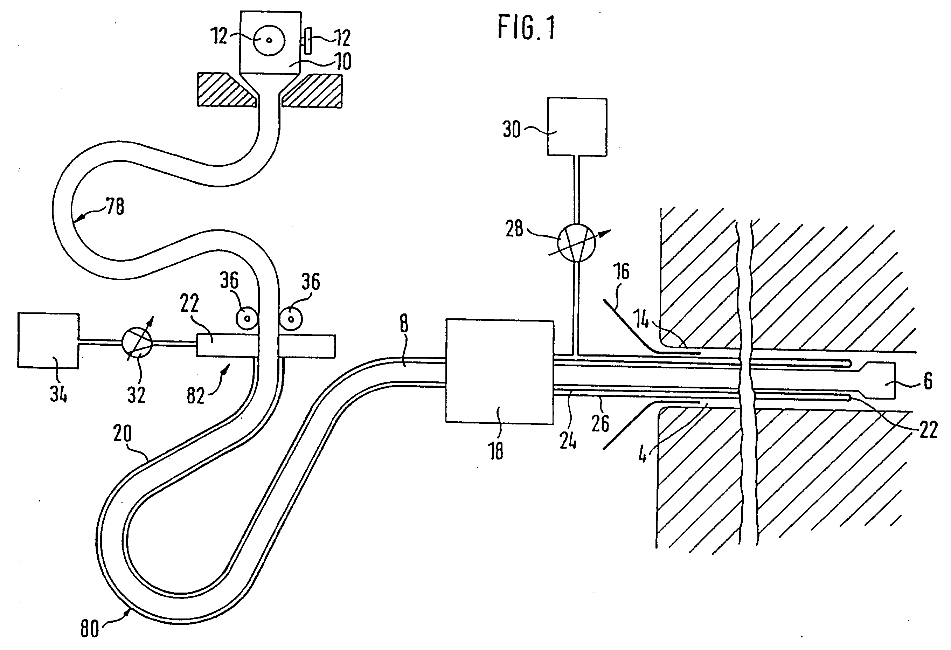

Fig. 1 zeigt die Vorrichtung in einem Zustand, in der ein Endoskop 2 bereits ein Stück weit in den Darm 4 eines Patienten hineinbewegt worden ist. Das Endoskop 2 besteht im wesentlichen aus einem Distalende 6, einem Endoskopschaft 8 und einem Bedienungsende 10. Der Endoskopschaft 8 ist etwa 3200 mm lang und hat einen Durchmesser von 9 bis 13,5 mm. Das Distalende 6 hat einen etwas größeren Durchmesser. An dem Bedienende sind schematisch drehbare Betätigungsscheiben 12 zum Betätigen von Bedienungsdrähten zum Abwinkeln des Distalendes 6 eingezeichnet. In den Anus 14 ist ein Tubus 16 eingeführt, um zu verhindern, daß der Patient den Anus-Schließmuskel schließt.

In einem Abstand von etwa 10 bis 20 cm vom Anus 14 befindet sich eine erste Antriebseinrichtung 18. Ein Stülpschlauch 20 ist mit seinem hinteren Ende an einem plattenartigen Teil 22 befestigt und umgibt von hier aus bis nahe zum vorderen Ende des Endoskops 2 den Endoskopschaft 8. Nahe dem vorderen Ende des Endoskops 2 ist der Stülpschlauch 20 um 180° nach außen und dann wieder zurück umgestülpt und führt von dort wieder zurück zu der ersten Antriebseinrichtung 18. Das vordere Ende des Stülpschlauchs 20 wird Umstülpbereich 22 genannt. Zwischen dem Umstülpbereich 22 und der ersten Antriebseinrichtung 18 weist der Stülpschlauch 20 somit einen inneren Bereich 24 und einen äußeren Bereich 26 auf.

Der Raum zwischen dem inneren Bereich 24 und dem äußeren Bereich 26, rechts in Fig. 1 abgeschlossenen durch den Umstülpbereich 22 und links in Fig. 1 zeitweise abgeschlossen innerhalb der ersten Antriebseinrichtung 18, kann mittels einer Pumpe 28 mit Flüssigkeitsdruck beaufschlagt werden. Die Pumpe 28 saugt die Flüssigkeit aus einem Vorratsbehälter 30 an. Als Flüssigkeit kann Wasser mit zugesetztem Benetzungsmittel verwendet werden.

Zwischen der ersten Antriebseinrichtung 18 und der Platte 22 ist der Endoskopschaft 8 mit dem umgebenden Schlauch 20 zu einer ausgebuchteten Form gelegt. Im Bereich der Platte 22 kann Schmiermittel in den Raum zwischen dem Endoskopschaft 8 und dem Schlauch 20 eingepreßt werden. In Fig. 1 ist eine Schmiermittelpumpe 32 und ein zugeordneter Vorratsbehälter 34 für Schmiermittel eingezeichnet. Bei dem Schmiermittel kann es sich zum Beispiel um Pflanzenöl handeln.

Auf der dem Schlauch 20 abgewandten Seite der Platte 22 ist ein Paar von Antriebsrädern 36 vorgesehen, die mit ihrem rinnenförmigen Außenumfang von zwei Seiten her mit dem Endoskopschaft 8 in reibschlüssigem Eingriff sind. Diese Antriebsräder 36 bilden zusammen mit einem nicht eingezeichneten Elektromotor eine zweite Antriebseinrichtung 72.

Im Längenbereich zwischen den Antriebsrädern 36 und dem Bedienungsende 10 des Endoskops 2 ist der Endoskopschaft 8 ebenfalls in eine ausgebuchtete Form gelegt.

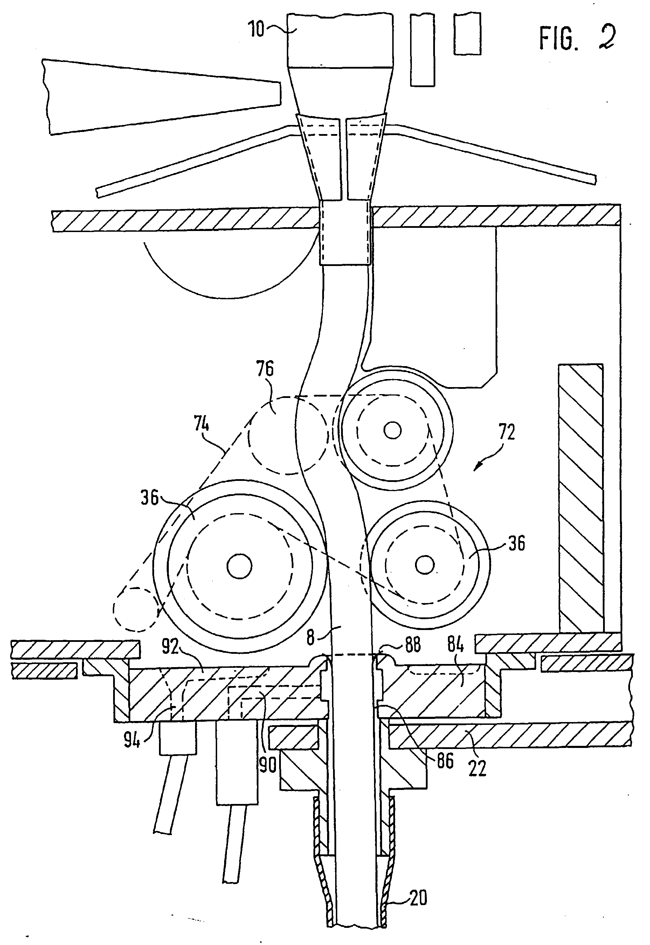

Fig. 2 veranschaulicht konkreter die zweite Antriebseinrichtung 72, deren Antriebsräder 36 bereits im Zusammenhang mit Fig. 1 beschrieben worden sind. Die Antriebsräder 36 stehen über eine Kette 74 mit einem antreibenden Kettenrad 76 in Verbindung, welches seinerseits von einem nicht eingezeichneten Elektromotor in beiden Drehrichtungen antreibbar ist. Während einer Vorwärtsbewegung des Endoskopschafts 8 zusammen mit dem inneren Bereich 24 des Stülpschlauchs 20 verkleinert sich die Ausbuchtung 80 zwischen der ersten Antriebseinrichtung 18 und der Platte 22. Am Ende des Hineinbewegens des Endoskops 2 in den Darm 4 ist die Ausbuchtung 80 praktisch aufgebraucht; es befinden sich etwa 1500 mm Endoskoplänge in der Ausbuchtung 78.

In Fig. 2 erkennt man ferner weitere Details der Schmiermittel-Einpreßeinrichtung 82 (vergleiche Fig. 1). Oberhalb der Platte 22 ist ein plattenartiges Schmiermittel-Zuführbauteil 84 angeordnet. Das Bauteil 84 weist eine Durchgangsöffnung 86 auf, durch die der Endoskopschaft 8 hindurchgeführt ist. In ihrem oberen Bereich ist die Durchgangsöffnung 86 auf, durch die der Endoskopschaft 8 hindurchgeführt ist. In ihrem oberen Bereich ist die Durchgangsöffnung 86 mittels einer Lippendichtung 88 gegenüber dem Endoskopschaft 8 abgedichtet. Im darunter befindlichen Bereich hat die Durchgangsöffnung 86 einen deutlich größeren Innendurchmesser als der Außendurchmesser des Endoskopschafts 8. Dort mündet ein Zuführkanal 90 für das Schmiermittel, der an die Schmiermittelpumpe 32 angeschlossen ist. An seiner Oberseite ist das Bauteil 84 mit einer umlaufenden Vertiefung 92 versehen, von der ein Rücklaufkanal 94 für Schmiermittel, welches zwischen der Dichtung 88 und dem Endoskopschaft 8 möglicherweise nach oben austritt, zurück zu dem Vorratsbehälter 34 führt.

Schmiermittel, welches durch den Kanal 90 unter Druck in den Bereich zwischen der Durchgangsöffnung 86 und dem Endoskopschaft 8 gelangt, breitet sich zwischen dem inneren Bereich 24 des Stülpschlauchs 20 und dem Endoskopschaft 8 auf die gesamte Länge aus. Überschußmengen des Schmiermittels treten im Bereich des Umstülpbereichs 22 des Stülpschlauchs 20 in den Darm 4 aus. Die Schmiermitteleinpreßeinrichtung 82 kann so gesteuert sein, daß jeweils bei Beginn einer Zurückbewegung des Endoskopschafts 8 mittels der Antriebsräder 36 ein kurzer Schmiermittelstoß abgegeben wird.

Mit der zweiten Antriebseinrichtung 72 kann man den Endoskopschaft 8 wahlweise vorwärts oder rückwärts über eine längere Bewegungsstrecke bewegen. Ein typischer Anwendungsfall für diese Bewegung ergibt sich, wenn das Distalende 6 des Endoskops 2 die innerste Dickdarmbiegung passiert hat. Dann schließt sich ein relativ gerader Dickdarmabschnitt an, den man mittels Ausfahrens des Endoskops ohne Mitnahme des inneren Bereichs 24 des Stülpschlauchs 20 explorieren kann. Es gibt Endoskope, deren Distalende man mit den beschriebenen Drahtzügen um bis zu 160° abwinkeln kann, und bei gegenüber dem Umstülpbereich 22 vorgefahrenem Distalende 6 kann man also quasi auch nach rückwärts blickend den Darm explorieren. Der Stülpschlauch 20 kann aus Silikon bestehen und kann beidseitig z. B. mit Polytetrafluorenethylen beschichtet sein.

Außerdem kann die gesamte Vorrichtung höhenverstellbar sein, damit man die Höhe des Endoskopschafts 8 an die Höhe, die der Anus des für die Untersuchung gelagerten Patienten hat, anpassen kann. Es versteht sich, daß die Lage des Patienten relativ zu der Vorrichtung im Verlauf der Untersuchung beibehalten werden sollte; insbesondere sollte der Abstand zwischen dem Anus 14 und der ersten Antriebseinrichtung 18 konstant sein.

Die beschriebene Vorrichtung hat für den untersuchenden Arzt ferner den Vorteil, daß er mit einem Bedienende 10 des Endoskops 2 arbeiten kann, welches Bedienende 10 stationär auf der Vorrichtung angebracht ist. Das Hineinbewegen und das Herausbewegen des Endoskopschafts 8 ist ohne Einfluß auf die stationäre Position des Bedienendes 10. Der Arzt kann das Hineinbewegen und das Herausbewegen des Endoskopschafts bequem durch elektrische Schalter an der Vorrichtung steuern. Vor Biegungen des Darms winkelt er das Distalende 6 des Endoskops 2 so ab, daß dieses im wesentlichen in Richtung des vor dem Distalende 6 liegenden Darmverlaufs weist. Alternativ ist es z. B. möglich, den Stülpschlauch 20 und den Endoskopschatt 8 allmählich immer weiter in den Darm 4 hineinzuschieben in einem Zustand, bei dem das vordere Ende des Distalendes 6 geringfügig hinter (also in Fig. 1 links von) dem Umstülpbereich 22 zurück ist. Dann läuft dem vorderen Ende des Distalendes 6 stets der Umstülpbereich 22 geringfügig voraus, so daß während des Hineinschiebens das Distalende 6 nie in Gefahr ist, mit der Darmwand in Berührung zu kommen.

Aufgrund der vorangegangenen Beschreibung ist klar, daß bei dem beschriebenen Ausführungsbeispiel die zweite Antriebseinrichtung 72 zugleich diejenige Antriebseinrichtung darstellt, mit welcher der Endoskopschaft 8 bedarfsweise ein größeres Stück vorwärts oder rückwärts bewegt werden kann, unabhängig von dem Stülpschlauch 20.

Aus der vorstehenden Beschreibung ist ferner ersichtlich, daß streng genommen jeweils der Endoskopschaft 8 mitsamt dem Distalende 6 vorwärts- und rückwärtsbewegt wird, nicht jedoch das Bedienende 10.

Anschließend wird die Bewegungs-Funktionsweise der Vorrichtung beschrieben:

Man kann zu denjenigen Zeitphasen, in denen die erste Antriebseinrichtung 18 eine Bewegung des Stülpschlauchs 20 und des Endoskopschafts 8 in eine erste Richtung leistet, z. B. mittels der zweiten Antriebseinrichtung 72 dafür sorgen, daß der Endoskopschaft 8 mit der halben Geschwindigkeit oder um die halbe Strecke in der ersten Richtung bewegt wird. Mit anderen Worten: Der Endoskopschaft 8 vollführt bei Bewegungen in der ersten Richtung eine zurückbleibende Gleitbewegung relativ zu dem Stülpschlauch 20. Die Voraussetzung hierfür, daß nämlich die Reibung zwischen dem Schlauchstück 46 und dem Außenumfang des Stülpschlauchs 20 in der ersten Antriebseinrichtung 18 die Reibung größer ist als zwischen dem Endoskopschaft 8 und dem Stülpschlauch 20, ist gegeben. Wegen der sich allmählich aufbrauchenden Ausbuchtung 80 wird der Endoskopschaft 8 durch die Antriebsräder 36 dort mit der gleichen Geschwindigkeit rückwärts gezogen wie sich der vordere Teil des Endoskopschafts 8 vorwärts in den Darm 4 hineinbewegt.

Man kann sogar so weit gehen, die Bewegung des Endoskopschafts 8 und des Stülpschlauchs 20 in die erste Richtung kontinuierlich oder alternativ intermittierend durchzuführen. Wenn man z. B. die erste Antriebseinrichtung mit einem in reibschlüssigem Eingriff mit dem Stülpschlauch 20 insbesondere dessen inneren Wandbereich 24 stehendes, elektromotorisch betriebenes Antriebsräderpaar versieht und mit einer weiteren Antriebseinrichtung beispielsweise der zweiten Antriebseinrichtung 72 dafür sorgt, daß sich der Stülpschlauch 20 bzw. der innere Bereich 24 des Stülpschlauchs 20 mit der doppelten Geschwindigkeit wie der Endoskopschaft 8 in die erste Richtung bewegt, hat man das Ziel erreicht, den Stülpschlauch 20 und den Endoskopschaft 8 gemeinsam passen im Darm 4 vorwärts zu bewegen.

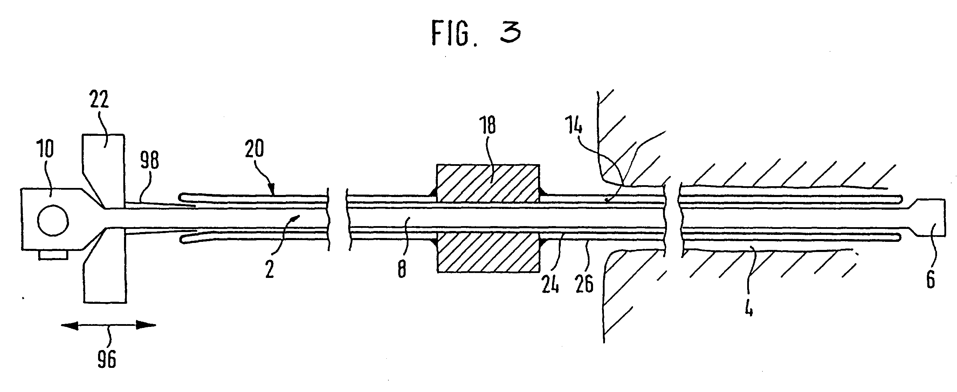

Die Ausführungsform gemäß Fig. 3 unterscheidet sich von der bisher beschriebenen Ausführungsform gemäß Fig. 1 und 2 im wesentlichen dadurch, daß der Stülpschlauch 20 in seinem links von der ersten Antriebseinrichtung 18 befindlichen Bereich nach außen umgestülpt und mit seinem dortigen Ende an der ersten Antriebseinrichtung 18 befestigt ist. Zwischen der ersten Antriebseinrichtung 18 und der Platte 22 hat der Endoskopschaft 8 mitsamt dem Stülpschlauch 20 einen geraden Verlauf statt der Ausbuchtung 80. Das Bedienende 10 des Endoskops 2 sitzt unmittelbar an der Platte 22. Die Platte 22 ist in Längsrichtung des Endosopschafts 8 bewegbar, s. Pfeil 96. Ein Beispiel hierfür ist die Anbringung der Platte 22 auf einem in Horizontalrichtung verfahrbaren Schlitten, wobei vorzugsweise eine nicht eingezeichnete, zweite Antriebseinrichtung vorgesehen ist, um die Platte 22 mindestens in Fig. 3 nach links gerichtet anzutreiben.

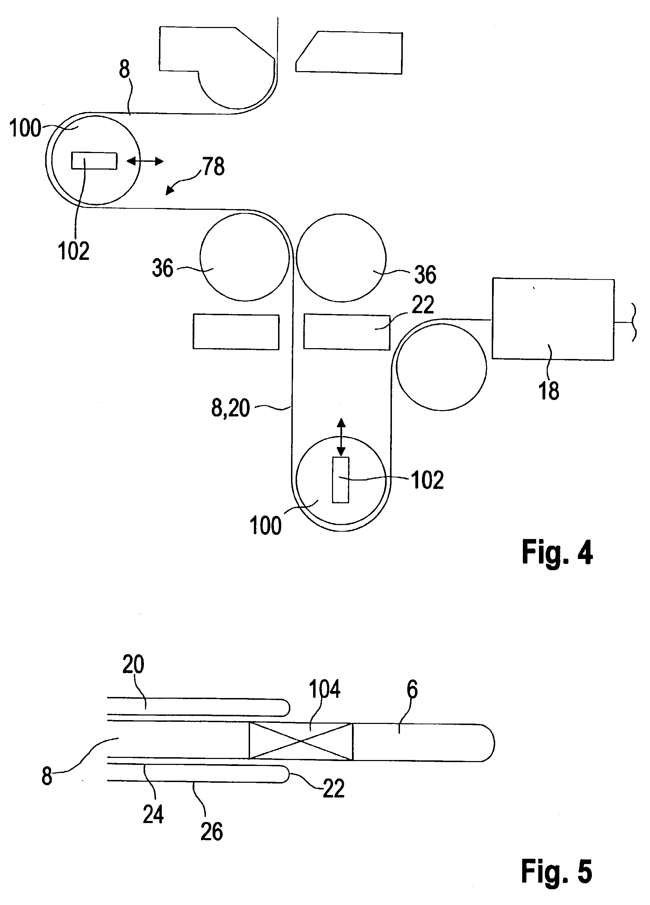

Beim Hineinbewegen des Endoskopschafts 8 in den Darm 4 verkürzt sich derjenige Abschnitt des Stülpschlauchs 20, der sich links von der ersten Antriebseinrichtung 18 befindet, analog dazu, wie sich derjenige Abschnitt des Stülpschlauchs 20, der sich rechts von der ersten Antriebseinrichtung 18 befindet, verlängert. Mit anderen Worten: Der innere Bereich 24 des Stülpschlauchs 20 wird durch die erste Antriebseinrichtung 18 hindurchbewegt. Die Bewegungen des Endoskopschafts 8 in die zweite Richtung werden durch angetriebene Bewegungen der Platte 22 nach links in Fig. 4 erzeugt. Es ist selbstverständlich möglich, die Ausführungsform gemäß Fig. 3 so zu konstruieren, daß die Bewegungen in die erste Richtung und/oder in die zweite Richtung kontinuierlich durchgeführt werden, wie weiter vorne im Zusammenhang mit der ersten Ausführungsform beschrieben.

In Fig. 3 erkennt man ferner ein ringförmiges, schürzenartiges Teil 98, welches nach rechts weisend an der Platte 22 befestigt ist und in den Raum zwischen dem inneren Bereich 24 des Stülpschlauchs 20 und dem Endoskopschaft 8 ragt, wobei der innere Bereich 24 des Stülpschlauchs 20 möglichst gut abgedichtet mit dem Teil 98 in Kontakt ist. Aus dem Raum innerhalb des schürzenartigen Teils 98 kann Schmiermittel in den Raum zwischen dem inneren Bereich 24 des Stülpschlauchs 20 und der Außenoberfläche des Endoskopschafts 8 eingepreßt werden, analog wie im Zusammenhang mit der ersten Ausführungsform geschildert.

Die Ausführungsform gemäß Fig. 3 hat gegenüber der ersten Ausführungsform den Vorteil, daß man mit einem handelsüblichen Koloskop mit einer Länge von gut 1500 mm arbeiten kann. Am Anfang befindet sich dieses Endoskoplänge zwischen dem Anus 14 und der Platte 22. Am Ende des Hineinbewegens des Endoskopschafts 8 in den Darm 4 ist die Platte 22 an die erste Antriebseinrichtung 18 herangerückt; die Koloskoplänge befindet sich zwischen der ersten Antriebseinrichtung 18 und dem Explorationsende im Darm 4.

Am Ausführungsbeispiel gemäß Fig. 3 wird besonders deutlich, daß bei Ausführungsformen der Vorrichtung ohne Vorrat von Schlauch 20 und Endoskopschaft 8 (Vorrat 80) bei der vorstehend beschriebenen Bewegungsfunktionsweise eine rückwärts gerichtete, hemmende Kraft auf den Endoskopschaft 8 ausgeübt werden muß, um die Bewegungsgeschwindigkeit des vorderen Teils des Endoskopschafts 8 auf im wesentlichen die Hälfte der Bewegungsgeschwindigkeit des vorderen Teils des inneren Bereichs 24 des Schlauchs 20 einzustellen. Dies wird bei der Ausführungsform gemäß Fig. 3 durch eine Bewegung der Platte 22 nach rechts mit entsprechender Geschwindigkeit geleistet. Insofern spricht man bei der erfindungsgemäßen Bewegungsfunktionsweise sinnvoller Weise von einer Bewegungsbestimmungseinrichtung als von einer zweiten Antriebseinrichtung.

Hierbei ist es bei der erfindungsgemäßen Bewegungsfunktionweise günstig, wenn die Anpreßkräfte zwischen den Antriebsrädern der Antriebeinrichtung 18 und dem inneren Wandbereich 24 des Stülpschlauchs 20 tendenziell so niedrig sind, um die Relativ-Gleitbewegung zwischen dem schnelleren Schlauch 20 und dem langsameren Endoskopschaft 8 nicht übermäßig zu behindern. Andererseits ist es jedoch günstig, für einen recht hohen Reibungskoeffizienten zwischen den Antriebsrädern der Antriebseinrichtung 18 und dem Außenumfang des inneren Bereichs 24 des Schlauchs 20 zu sorgen, um eine genügend große Antriebskraft auf den Stülpschlauch und den Endoskopschaft aufbringen zu können.

Versuche haben ergeben, daß es durchaus möglich ist, einerseits genügend Vortriebskraft auf den inneren Bereich 24 des Schlauchs 20 auszuüben und durch den Mitnahmeeffekt zwischen dem inneren Bereich 24 des Schlauchs 20 und dem Endoskopschaft 8 Vortriebskraft auf den Endoskopschaft 8 auszuüben und andererseits mittels der Bewegungsbestimmungseinrichtung 72 eine derartige rückwärtsgerichtete Kraft auf den Endoskopschaft auszuüben, daß er mit dem beschriebenen Geschwindigkeitsverhältnis gegenüber dem inneren Bereich 24 des Schlauchs 20 zurückbleibt.

Der Druck im Raum zwischen dem äußeren Bereich 26 und dem inneren Bereich 24 des Schlauchs 20 beträgt vorzugsweise 0,4 bis 1,2 bar, besonders bevorzugt 0,6 bis 0,8 bar. Der Druck, mit dem das Schlauchstück 46 nach innen gepreßt wird, beträgt vorzugsweise 0,8 bis 1,6 bar, besonders bevorzugt 1 bis 1,3 bar, wobei dieser Druck zur Sicherung der dortigen Abdichtung etwas höher sein sollte als der Druck zwischen dem inneren und dem äußeren Bereich des Schlauchs 20. Der Druck, mit dem Schmiermittel eingepreßt wird, beträgt vorzugsweise 0,2 bis 0,8 bar. Sämtliche, in der Anmeldung genannten Druckwerte bedeuten Überdruck über dem athmosphärischem Druck. Die Wandstärke des Schlauchs 20 beträgt vorzugsweise 0,8 bis 1,2 mm, besonders bevorzugt 0,9 bis 1,1 mm.

Es versteht sich, daß man sinnvollerweise die Relation der Bewegungsgeschwindigkeit der Antriebseinrichtung 18 und die durch die Bewegungsbestimmungseinrichtung 72 definierte Bewegungsgeschwindigkeit des Endoskopschafts 8 durch eine geeignete Steuerung so aufeinander abstimmt, daß sich das beschriebene Geschwindigkeisverhältnis ergibt. Eine geeignete Steuerung hierfür ist mit dem Wissen des Durchschnittsfachmanns erstellbar.

Anhand von Fig. 4 wird veranschaulicht, daß man für die Ausbuchtung 80 und für die Ausbuchtung 78 jeweils ein Vorratsstraffungselement in Gestalt einer Umlenkrolle 100 vorsehen kann. Jede der Umlenkrollen 100 steht unter der Wirkung einer nicht eingezeichneten Feder derart, daß die Ausbuchtung 80 bzw. 78 straff gehalten wird. Ferner ist für jede der Umlenkrollen 100 ein schematisiert eingezeichneter Wegaufnehmer 102 vorgesehen. Durch Vergleich der Signale der beiden Wegaufnehmer 102 läßt sich auch ermitteln, ob das Distalende 6 des Endoskopschafts 8 sich ordnungsgemäß etwas vor dem Umstülpbereich 22 des Schlauchs 20 befindet. Wenn eine Abweichung von der Soll-Relativlage ermittelt wird, kann korrigierend eingegriffen werden, z. B. durch geringfügige Erhöhung oder Erniedrigung der Drehzahl der Antriebsräder 36.

Anhand von Fig. 5 wird eine unmittelbarere Ermittlung der Soll-Relativlage des Distalendes 6 des Endoskopschafts 8 relativ zu dem Umstülpbereich 22 des Schlauchs 20 beschrieben. Ein kurzes Stück zurückgesetzt gegenüber dem vorderen Ende des Endoskops ist außenseitig auf diesem ein an sich bekannter Folienschalter 104 befestigt. Wenn der Folienschalter 104 durch den inneren Bereich 24 des Schlauchs 20 druckbeaufschlagt und dadurch geschlossen wird, wird ein Signal abgegeben, welches bedeutet, daß der Endoskopschaft 8 nicht noch weiter gegenüber der Vorwärtsbewegung des Umstülpbereichs 22 zurückbleiben soll. Aufgrund dieses Signals läßt sich z. B. die Bewegungsbestimmungseinrichtung geringfügig in ihrer Geschwindigkeit verstellen in dem Sinne, daß die Bewegungsgeschwindigkeit des Endoskopschafts 8 etwas größer als bisher gemacht wird. Am vorteilhaftesten ist es, einen Doppelschalter mit zwei in Längsrichtung beabstandeten Schaltstellen vorzusehen. Die Soll-Relativlage des Distalendes 6 ist erreicht, wenn die hintere Schaltstelle geschlossen ist und die vordere Schaltstelle geöffnet ist.

## Patentansprüche

1. Vorrichtung zum Bewegen eines Endoskopschafts (8) längs eines kanalartigen Hohlraums (4) mit einem zumindest eine Teillänge des Endoskopschafts (8) aufnehmenden Stülpschlauch (20), der beim Einsatz einen relativ zu der Hohlraumwand ruhenden, vorderen äußeren Wandbereich (26) hat, der an seinem einen Ende an einer Antriebseinrichtung (18) zum Fortbewegen des Endoskopschafts (8) fixiert und an einem distalen Endabschnitt (6) des Endoskopschafts (8) zu einem inneren Wandbereich (24) umgestülpt ist, **dadurch gekennzeichnet, daß**
die Antriebseinrichtung (18) Antriebsräder hat, die auf einen Abschnitt des inneren Wandbereichs (24) des Stülpschlauchs (20) eine radiale Anpreßkraft ausüben, wodurch über den inneren Wandbereich (24) eine Antriebskraft auf den Endoskopschaft (8) für dessen kontinuierliche Bewegung übertragbar ist, dessen
Bewegungsgeschwindigkeit mittels einer auf den Endoskopschaft (8) einwirkenden Bewegungsbestimmungseinrichtung (22, 72) bezüglich der Bewegungsgeschwindigkeit des inneren Wandbereichs (24) derart regelbar ist, daß die Bewegungsgeschwindigkeit des inneren Wandbereichs (24) im wesentlichen doppelt so groß ist, wie die Bewegungsgeschwindigkeit des Endoskopschafts (8).

2. Vorrichtung nach Anspruch 1, **dadurch gekennzeichnet, daß** die Bewegungsbestimmungseinrichtung (72) derart ausgebildet ist, daß sie auf den Endoskopschaft (8) eine der ersten Antriebseinrichtung (18) entgegenwirkende Antriebs- bzw. Bremskraft kontinuierlich aufbringt.

3. Vorrichtung nach Anspruch 2, **dadurch gekennzeichnet, daß** die Bewegungsbestimmungseinrichtung (72) eine Anzahl von elektromotorisch betreibbaren Antriebsrädern (36) hat, die unmittelbar auf den Endoskopschaft (8) aufgedrückt sind.

4. Vorrichtung nach einem der vorstehenden Ansprüche, dadurch gekennzeichnet, daß der innere Wandbereich (24) an einem zum distalen Endabschnitt (6) des Endoskopschafts (8) gegenüberliegenden hinteren Endabschnitt zu einem hinteren äußeren Wandbereich umgestülpt ist, der zur Antriebseinrichtung (18) zurückgeführt und an dieser fixiert ist.

5. Vorrichtung nach Anspruch 4, **dadurch gekennzeichnet, daß** die Bewegungsbestimmungseinrichtung (72) eine auf dem Endoskopschaft (8) vor dem hinteren Endabschnitt des Stülpschlauchs (20) fixierte Platte (22) hat, die vorzugsweise auf einem verfahrbaren Schlitten gelagert ist.

6. Vorrichtung nach einem der vorstehende Ansprüche, **gekennzeichnet durch** eine Druckfluid-Zuführeinrichtung (28), mit der Druckfluid in einen Raum zwischen dem äußeren Wandbereich (26) und dem inneren Wandbereich (24) des Stülpschlauchs (20) zuführbar ist.

7. Vorrichtung nach einem der vorstehenden Ansprüche, **gekennzeichnet durch** eine Schmiermittel-Einpreßeinrichtung (82), die Schmiermittel in einen Spalt zwischen dem Endoskopschaft (8) und dem inneren Wandbereich (24) einpreßt, um eine Gleitbewegung des Endoskopschafts (8) relativ zum inneren Wandbereich (24) zu ermöglichen.

8. Vorrichtung nach Anspruch 7, **dadurch gekennzeichnet, daß** als Schmiermittel ein Pflanzenöl verwendet ist.

9. Endoskopiegerät mit einem Endoskopschaft (8), **gekennzeichnet durch** eine Vorrichtung zum Bewegen des Endoskopschafts (8) gemäß einem der Ansprüche 1 bis 8.

## Claims

1. A device for moving an endoscope shaft (8) along a canal-shaped cavity (4), said device comprising a reversible tube (20) which receives at least a partial length of said endoscope shaft (8) and which, upon use of said device, has a front outer wall area (26) that remains stationary with respect to the cavity wall, said front outer wall area (26) being fixed at its one end to a driving means (18) for moving said endoscope shaft (8) and being reversed at a distal end portion (6) of said endoscope shaft (8) to form an inner wall area (24), **characterized in that**
said driving means (18) has driving wheels which apply a radial pressing force on a portion of said inner wall area (24) of said reversible tube (20) whereby a driving force can be transmitted via said inner wall area (24) to said endoscope shaft (8) for continuos movement thereof, whose rate of motion is, by means of a motion determining means (22, 72) acting on said endoscope shaft (8), controllable with respect to the rate of motion of said inner wall area (24) such that the rate of motion of said inner wall area (24) is substantially double the rate of motion of said endoscope shaft (8).

2. A device according to claim 1, **characterized in that** said motion determining means (72) is designed such that it continuously applies to said endoscope shaft (8) a driving or braking force counteracting said first driving means (18).

3. A device according to claim 2, **characterized in that** said motion determining means (72) comprises a number of driving wheels (36) drivable by an electric motor which are directly pressed onto said endoscope shaft (8).

4. A device according to one of the preceding claims, **characterized in that** said inner wall area (24) is, at a rear end portion opposite to said distal end portion (6) of said endoscope shaft (8), reversed to form a rear outer wall area which is led back to said driving means (18) and is fixed to the same.

5. A device according to claim 4, **characterized in that** said motion determining means (72) includes a plate (22) which is fixed on said endoscope shaft (8) in front of said rear end portion of said reversible tube (20) and is preferably supported on a movable slide.

6. A device according to one of the preceding claims, **characterized by** a pressure fluid supply means (28) by means of which pressure fluid can be supplied into a space between said outer wall area (26) and said inner wall area (24) of said reversible tube (20).

7. A device according to one of the preceding claims, **characterized by** a lubricant injecting means (82) injecting lubricant into a gap between said endoscope shaft (8) and said inner wall area (24) so as to enable a sliding movement of said endoscope shaft (8) relative to said inner wall area (24).

8. A device according to claim 7, **characterized in that** a vegetable oil is used as lubricant.

9. An endoscopic device comprising an endoscope shaft (8), **characterized by** a device for moving said endoscope shaft (8) according to one of claims 1 to 8.

## Revendications

1. Dispositif de déplacement d'une tige d'endoscope (8) le long d'une cavité (4) en forme de canal, avec un tuyau de recouvrement (20) recouvrant au moins une partie de la longueur de la tige (8), ayant, lorsqu'il est utilisé, une zone de paroi (26) avant extérieure immobile par rapport à la paroi de la cavité, qui est fixé à ses extrémités à un dispositif d'entraînement (18) pour le déplacement de la tige d'endoscope (8) et qui est retroussé en une zone de paroi interne (24) à une section d'extrémité distale (6) de la tige d'endoscope (8), caractérisé en ce que le dispositif d'entraînement (18) comporte des roues d'entraînement qui exercent sur une section de la zone de paroi interne (24) du tuyau de recouvrement (20) une force de compression radiale, par laquelle, à travers la zone de paroi interne (24) est transmise une force d'entraînement sur la tige d'endoscope (8) pour son déplacement en continu, dont la vitesse de mouvement peut âtre réglée au moyen d'un dispositif de détermination du mouvement (22, 72) agissant sur la tige d'endoscope (8), par rapport à la vitesse dc déplacement de la zone de paroi interne (24), de telle façon que la vitesse de déplacement de la zone de paroi interne (24) est essentiellement le double de celle de la tige d'endoscope (8).

2. Dispositif selon la revendication 1, caractérisé en ce que le dispositif de détermination du mouvement (72) est réalisé de telle sorte qu'il applique en continu sur la tige d'endoscope (8) une force d'entraînement ou de freinage antagoniste au premier dispositifd'entraînement (18).

3. Dispositif selon la revendication 2, caractérisé en ce que le dispositif de détermination du mouvement (72) comporte une pluralité de roues d'entraînement (36) entraînées électriquement, qui sont pressées directement contre la tige d'endoscope (8).

4. Dispositif selon l'une des revendications précédentes, caractérisé par le fait que la roue de paroi interne (24) est retroussée à une section terminale arrière située en face dc la section terminale distale (6) de la tige d'endoscope (8), en une zone de paroi externe arrière qui est ramenée vers le dispositif d'entraînement (18) et lui est fixé.

5. Dispositif selon la revendication 4, caractérisé par le fait que le dispositif de détermination du mouvement (72) comporte une plaque (22) fixée sur la tige d'endoscope (8) avant la section terminale arrière du tuyau de recouvrement (20), qui est montée avantageusement sur un chariot coulissant.

6. Dispositif selon l'une des revendications précédentes, caractérisé par un dispositif d'alimentation on fluide de pression (28) au moyen duquel du fluide est amené dans un espace entre la zone de paroi extérieure (25) et la zone de paroi intérieure (24) du tuyau de recouvrement (20).

7. Dispositif selon l'une des revendications précédentes, caractérisé par un dispositif d'injection de lubrifiant (82) qui injecte sous pression du lubrifiant dans un intervalle entre la tige d'endoscope (8) et la zone de paroi interne (24) pour permettre un mouvement de glissement de la tige d'endoscope (8) par rapport à la zone de paroi interne (24).

8. Dispositif selon la revendication 7, caractérisé par le fait que le lubrifiant est une huile végétale.

9. Appareil d'endoscopie avec une tige d'endoscope (8), caractérisé par un dispositif pour mouvoir la tige d'endoscope (8) selon l'une des revendications 1 à 8.
